# EUROPÄISCHE PATENTSCHRIFT

(11) **EP 1 349 505 B1**
(45) Veröffentlichungstag und Bekanntmachung des Hinweises auf die Patenterteilung: **19.11.2008**
(21) Anmeldenummer: 02704579.8
(22) Anmeldetag: 11.01.2002
(51) Int. Cl.: A61B 17/16

(54) **BOHRER ZUM BOHREN IN KNOCHENGEWEBE**
DRILL FOR DRILLING IN BONE TISSUE
FORET POUR PERCER UN TISSU OSSEUX

(30) Priorität: 11.01.2001 DE 10101083
(43) Veröffentlichungstag der Anmeldung: 08.10.2003
(73) Patentinhaber: Kirsch, Axel, Dr., 70794 Filderstadt (DE)
(72) Erfinder: KIRSCH, Axel, 70794 Filderstadt (DE); DUERR, Walter, 75196 Remchingen (DE)
(74) Vertreter: Goddar, Heinz J.
(86) Internationale Anmeldenummer: PCT/DE2002/000072
(87) Internationale Veröffentlichungsnummer: WO 2002/054963

(56) Entgegenhaltungen:
- DE-A- 2 923 358
- FR-A- 1 072 069
- US-A- 6 139 214

## Beschreibung

Die Erfindung betrifft einen Bohrer zum Erzeugen von Bohrungen in Knochengewebe sowie die Verwendung eines zugehörigen Bohraufsatzes.

Derartige Bohrer mit einem Anschlußstück, die häufig noch eine Kühlvorrichtung im Inneren des Bohrers aufweisen, sind aus dem Stand der Technik sowie früheren Anmeldungen des Erfinders bekannt.

Insbesondere ist die deutsche Patentschrift DE 4103663 C2 zu nennen, in der bereits ein chirurgisches Handstück beschrieben ist, das einen Bohraufsatz mit einem rotationssymmetrischen zylindrischen Vorderteil und einem mehrkantigem Schaftabschnitt aufweist, der mit einer Schnellspanneinrichtung gehalten wird. Dieses Handstück weist ein Anschlussstück, in dem eine angetriebene Aufnahme vorgesehen ist, und ein Werkzeug auf, das mit der Aufnahme über eine Kupplungseinrichtung lösbar verbunden ist, wobei zumindest ein Abschnitt eines Schafts des Werkzeugs in Mehrkantform ausgebildet ist und formschlüssig in einem komplementär ausgebildeten Bereich der Aufnahme in Anschlussstück aufgenommen ist, und ein distaler, im wesentlichen zylindrischer Abschnitt des Werkzeugs, z.B. zum Bohren, vorhanden ist.

Die Verriegelung des Bohraufsatzes ist aber aufwendig und hat sich insgesamt als schwierig zu lösende Aufgabe herausgestellt. So schlägt beispielsweise das US-Patent 5,074,789 längliche Verriegelungselemente vor, die während der Benutzung frei an der Außenseite liegen und ein genau winkelrichtiges Einsetzen des Bohraufsatzes in das Anschlußstück vom Benutzer verlangen.

Wünschenswert wäre jedoch ein Bohraufsatz, der ohne vorherige Verdrehausrichtung in im wesentlichen jeder Verdrehstellung in die Aufnahme gesteckt werden könnte und dort stets sicher gehalten wird, ohne dass Beschädigungen oder Verunreinigungen an der Außenseite der Halteeinrichtung auftreten.

Bei innenliegenden Halterungen wurde zur Fixierung in axialer Richtung bisher meist eine Anzahl von Kugeln vorgesehen, die in eine umlaufende Nut im zylindrischen Abschnitt des Bohrerschafts eingriffen, wobei diese Nut an die Form und Größe der Kugeln angepaßt war, also im radialen Querschnitt teilkreisförmig war. Allerdings existieren verschiedene Kugelabmessungen, und der korrekte Sitz des Bohraufsatzes war bei Verwendung einer nicht den Kugeldimensionen entsprechenden Nut gefährdet.

Neben der Notwendigkeit für eine Vorrichtung zum Anpressen der Kugeln an den Bohrerschaft bildet diese Nut im Bohrerschaft - insbesondere bei der Anwesenheit einer zentralen Bohrung für Kühlmittel - eine Schwächung und bedeutete eine Verlängerung des Bohraufsatzes. Außerdem war sie in der Fertigung der Bohrerschäfte nur mit einen zusätzlichen Arbeitsschritt anbringbar.

FR-PS 1 072 069 offenbart ein zahnärztliches Winkelstück, bei dem ein Bohrwerkzeug über eine Kugelkupplung fixiert wird.

DE 29 23 358 A1 offenbart ein zahnärztliches Handstück, bei dem eine Führungsbuchse zur Führung des Schaftes eines einzusetzenden Werkzeugs in einem Hohlschaft mittels eines Federrings lösbar angeordnet ist. Diese Führungsbuchse kann durch ein spezielles Werkzeug, welches in Durchlässe des Hohlschafts eingeführt werden kann und den Federring konzentrisch zusammendrückt, gelöst werden.

Die Erfindung hat sich nun zur Aufgabe gestellt, einen Bohrer mit einer verbesserten Kupplungseinrichtung zum Erzeugen einer Bohrung im Knochengewebe zu schaffen, der sich mit einer einfacheren Schaftgeometrie realisieren läßt.

Erfindungsgemäß wird dies durch die Merkmale des Hauptanspruches gelöst. Die Unteransprüche geben vorteilhafte Merkmale der Erfindung wieder.

Vorteilhaft ist dabei, daß sowohl der Bohrerschaft, der an seinem Übergang von einem sechskantigen Abschnitt zu einem zylindrischen Abschnitt bereits eine teilkreisförmige Ringnut aus fertigungstechnischen Gründen aufweisen muß, wie auch die Aufnahme nun lediglich mit einer radialen im Querschnitt rechteckigen Fräsung auszubilden sind.

Eine weitere Fräsung zur Aufnahme der nach dem Stand der Technik notwendigen Haltekugeln, die eine Verlängerung des Schafts sowie eine Schwächung des Schafts an dieser Stelle bewirkte, kann entfallen.

Gleichzeitig wird durch die Vorsehung eines einfachen Federringes eine kostengünstig anbringbare, mechanisch sichere und von außen gegen Verschmutzungen geschützte und bei Ausbildung mit rotationssymmetrischen Nuten verdrehsichere Befestigung geschaffen.

Der Bohraufsatz kann insbesondere einen Sechskantabschnitt oder Achtkantabschnitt aufweisen.

Der Federring ist als geschlossener Innenring mit wenigstens zwei Federarmen ausgebildet, die vorzugsweise den Ring durch ihre Federkraft in eine exzentrische, verriegelnde Lage bringen, in der ein Teil des Innenumfangs, vorzugsweise im wesentlichen der halbe Innenumfang des Federrings in die Nut des Bohrerschafts einpaßt, während der Außenumfang des Federrings sowohl in der exzentrischen als auch in der konzentrischen Position noch in einer zweiten Nut des Anschlußstücks ruht. In einer zum Bohrerschaft konzentrischen Stellung kann der Bohrerschaft aber durch den Federring hindurch gelöst werden.

Die Ränder des Federrings können dabei in Richtung zu dem Mehrkantabschnitt des Bohraufsatzes hin abgeschrägt sein, um das Einsetzen des Bohraufsatzes zu erleichtern, während das Lösen des Bohraufsatzes über ein Druckelement erfolgt, das axial entlang des Anschlußstücks aus verlagerbar ist und den Federring entgegen der Kraft der Federarme in eine konzentrische Lage zum Bohrerschaft verschiebt, so daß die Nut des Bohrerschafts vom Federring verlassen wird und der Bohraufsatzabgezogen werden kann.

Hierzu wird vorgeschlagen, das Anschlußstück mit einem äußeren mit Federkraft vorgespannten verschieblichen Druckelement auszubilden, das an seinem dem Federring zugewandten Ende angeschrägt ist und im zurückgezogenen Zustand den Federring sich frei in seine exzentrische Lage verschieben läßt. Dieser zurückgezogene Zustand bildet die Ruhelage des Druckelements. In dieser Ruhelage ist das Druckelement außer Kontakt mit dem Federring.

Wenn das Druckelement in Richtung zum Bohrerkopf in eine zweite Position (Arbeitsstellung) verschoben wird, überführt das Druckelement den Federring in eine konzentrische Stellung gegen die Kraft der Federarme des Federrings , wobei der Federring die rechteckige Nut des Bohrerschafts verläßt. Hierzu wird vorgeschlagen, einen Teilkreisbereich des Federrings, insbesondere ein Segment gegenüber den Federarmen, mit einer geraden Kante, vorzugsweise im wesentlichen wie eine Sekante, auszubilden, entlang der das Druckelement vergleiten kann. Diese in der Ebene des Rings gerade Kante vergleitet mit der Schrägfläche an dem korrespondierend ausgebildeten Ende des Druckstücks.

Die zweite Nut in der Aufnahme weist zur lagerichtigen Aufnahme des Federrings daher eine annähernd ovale Form auf, in welcher der Federring sowohl in der exzentrischen als auch in der konzentrischen Position aufgenommen ist, die sich durch die vom Druckelement veranlaßte, im wesentlichen lineare Bewegung des geschlossenen Ringes ergeben.

Die Arme des Federrings verbleiben dabei stets in Kontakt mit der Innenfläche der zweiten Nut, vorzugsweise stets mit dem gleichen Bereich. Zum korrekten Einsetzen ist es daher denkbar, die zweite Nut mit einem Vorsprung zu versehen, der zwischen die beiden Endabschnitte der Federarme einpaßt, um den Ring schon während des Zusammenbaus vor einem verdrehten Einsetzen zu bewahren.

Die Erfindung stellt auch einen Bohrer gemäß dem Oberbegriff des Anspruchs 1 zur Verfügung, bei dem in dem Anschlußstück ein Ventil vorgesehen ist, welches entgegen einer Federkraft aus einer Ruhelage, in welcher es den Zufluß von Kühlmittel in die Kühlmittelzuführung der Aufnahme und damit in den Kühlmittelkanal des Bohraufsatzes verhindert, in eine Arbeitsstellung verschoben werden kann, in welcher der Zufluß von Kühlmittel freigegeben wird.

Dabei kann vorgesehen sein, daß das Ventil eine Anschlagfläche aufweist, an welcher, gegebenenfalls über zwischengeschaltete Elemente, der Schaft des Bohraufsatzes angreift, derart, daß mit dem Einführen des Bohrerschaftes in die Aufnahme der Ventilkopf des Ventils entgegen der Federkraft aus der Ruhelage in eine Arbeitsstellung überführt wird.

Insbesondere kann der Ventilkopf des Ventils durch den Bohraufsatz in einer Arbeitsstellung gehalten werden, wenn der Bohraufsatz in der Aufnahme bezüglich einer axialen Bewegung in der Aufnahme durch die Kupplungseinrichtung fixiert ist.

Es kann weiterhin vorgesehen sein, daß das Ventil sowie gegebenenfalls zwischengeschaltete Teile zwischen dem Ventil und dem Bohraufsatz in einer Hülse in axialer Richtung des Anschlußstücks verschieblich geführt sind. Die Feder kann von dem von dem Bohrer abgewandten Ende des Anschlußstücks aus auf den Ventilkopf einwirken.

Die Erfindung stellt weiterhin auch einen Bohrer gemäß dem Oberbegriff des Anspruchs 1 zur Verfügung, welcher dadurch gekennzeichnet ist, daß in der Aufnahme eine Rotationskupplung vorgesehen ist, deren drehbarer Teil, gegebenenfalls über geeignete Zwischenstücke, mit dem Ende des Bohraufsatzes gekoppelt ist, derart, daß sich dieses drehbare Teil mit dem Bohraufsatz dreht, und das feststehende Teil dieser Rotationskupplung in dem Anschlußstück fixiert ist, so daß es sich nicht drehen kann.

Dabei kann vorgesehen sein, daß die sich gegeneinander drehenden Teile der Rotationskupplung oder zumindest die Lagerflächen zwischen dem feststehenden Teil und dem rotierenden Teil der Rotationskupplung aus einem verschleißfesten Material, insbesondere einem Keramikmaterial oder aus einem Edelsteinmaterial, z. B. Rubin, bestehen.

Weitere Merkmale und Vorteile der Erfindung ergeben sich aus nachfolgender Beschreibung eines bevorzugten Ausführungsbeispiels der Erfindung anhand der beigefügten Zeichnung.

Dabei zeigt:
- Fig. 1: den Bohrer, wie er sich aus der Aufnahme und dem eingesetzten Bohreinsatz im zusammengebauten Zustand ergibt, .und
- Fig. 2: eine perspektivische Darstellung des in der Fig.1 im Schnitt dargestellten Federrings.

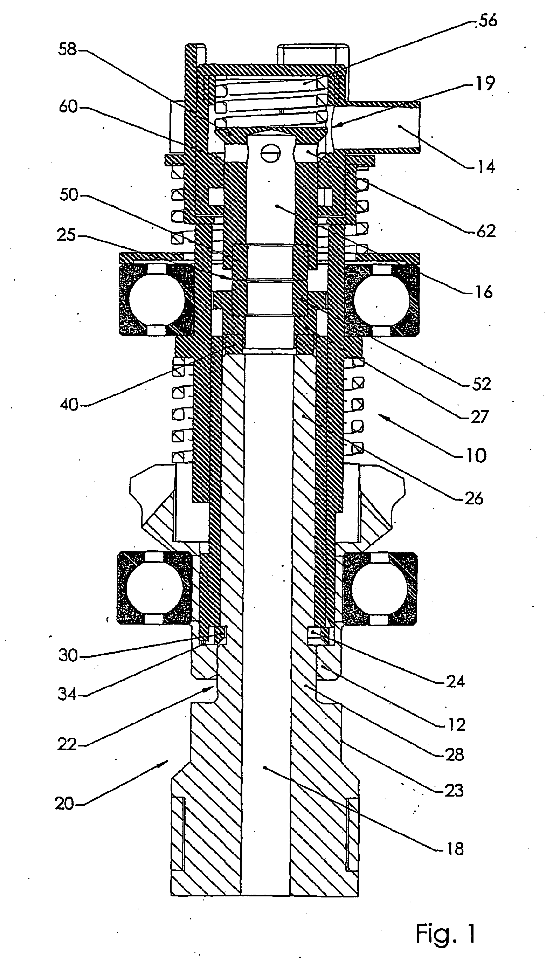

Fig. 1 zeigt einen Querschnitt durch ein erfindungsgemäßes Bohrgerät (Bohrer), das aus einem Anschlußstück 10 und einem eingeschobenen Bohraufsatz 20 besteht, der in eine Aufnahme 12 des Anschlußstückes 10 eingesetzt ist. Das Anschlußstück 10 umfaßt dabei einen Kühlmittelzufluß 14, der an eine externe, nicht gezeigte Kühlmittelversorgung, insbesondere einen Kühlmittelvorrat oder einen Anschluß zu einer Kühlmittelleitung, z. B. einer Wasserleitung, angeschlossen ist, und eine Kühlmittelzuführung 16 zur Abgabe des Kühlmittels an den Bohrerschaft 22, der mit einem Kühlmittelkanal 18 versehen ist.

Der Kühlmittelzufluß 14 ist dabei als Winkelstück von der Seite her kommend im Anschluß an einen Raum ausgebildet, in den hinein ein Ventil 19 gegen eine Feder beim Einsatz des Bohrerschafts 22 einfährt, um eine Fluidkommunikation mit dem Kühlmittelkanal 18 des Bohrerschafts über eine Kühlmittelzuführung 16 zu schaffen.

Dieses Kühlmittel dient bei Knochenbohrungen, wie sie im Dentalbereich zur Anwendung kommen, dazu, die Bohrerspitze zu kühlen, da eine Erhitzung des Knochengewebes beim Bohren zu einer Nekrose führen würde. Die Kühlflüssigkeit wird bei Bohrern meist über eine zentrale Kühlbohrung zugeführt, welche den Bohrer durchsetzt.

Die Kühlmittelzuführung zu dem Bohrerschaft 22 wird im einzelnen durch das bereits erwähnte Ventil 19, eine Rotationskupplung 25 und ein Zwischenstück 27 gebildet, welche jeweils eine zentrale Bohrung aufweisen, wobei die zentralen Bohrungen der genannten Teile miteinander fluchten, so daß ein durchgängiger Kanal zur Zuführung des Kühlmittels gebildet wird. Das Ventil 19 weist auf seiner zu dem Bohraufsatz hin gewandten Seite eine zentrale konzentrische Aufnahme für den feststehenden Teil 50 der Rotationskupplung 25 auf, der seinerseits fest, aber drehbar mit dem rotierenden Teil 52 der Rotationskupplung verbunden ist. Die Teile 50 und 52 bestehen aus einem hochverschleißfestem Material, z. B. aus Keramik oder Rubin, das eine hohe Lebensdauer besitzt. Das rotierende Teil 52 ist wiederum in einer Aufnahme des Zwischenstücks 27 angeordnet, das an seiner Unterseite eine Dichtung 40 aufweist.

Im Betrieb ist das Ventil 19 sowie das Teil 50 der Rotationskupplung 25 feststehend, während der rotierende Teil 52 der Rotationskupplung und das Zwischenstück 27, welche über die Dichtung 40 mit dem Bohraufsatz 20 gekoppelt sind, sich mit diesem drehen. Auf diese Weise ist das Lager zwischen zwei sich gegeneinander drehenden Flächen, der nach dem Stand der Technik zwischen dem Bohraufsatz und der Aufnahme vorgesehen war, von dem Bohraufsatz weg in die Aufnahme hinein verlegt. Dies hat den Vorteil, daß für die beiden Teile der Rotationskupplung 25 wesentlich beständigere Materialien verwendet werden können. Gleichzeitig werden die Funktionen der Dichtung und der drehfähigen Lagerung voneinander getrennt in der Rotationskupplung 25 und der Dichtung 40 realisiert, so daß für die beiden Funktionen jeweils das optimale Material verwendet werden kann.

Die ganze Einheit aus Ventil 19, Rotationskupplung 25 und Zwischenstück 27 ist entgegen der Kraft der Feder 56 aus einer Ruhestellung, in welcher der Ventilkopf 58 auf einem komplementären Ventilsitz 60 aufliegt, in der Einführrichtung des Bohraufsatzes in eine offene Stellung des Ventils 19 verlagerbar, in der zwischen dem Ventilkopf 58 und dem Ventilsitz 60 ein Spalt besteht, der mit einer in dem Ventilkopf ausgebildeten radialen Zuleitung 62 zu der Kühlmittelzuführung 16 kommuniziert. Wenn kein Bohraufsatz eingesetzt ist, drückt die Feder 56 den Ventilkopf 58 in den Ventilsitz 60, so daß ein Eintreten von Kühlmittel aus dem Kühlmittelzufluß 14 in die Kühlmittelzuführung 16 verhindert wird. Wenn der Bohraufsatz 20 in die Aufnahme 12 eingeschoben und in einer nachfolgend noch näher beschriebenen Weise verrastet wird, wird dagegen der Ventilkopf 58 entgegen der Kraft der Feder 56 nach oben gedrückt und gibt die radiale Zuleitung 62 des Ventilkopfs 58 frei, so daß Kühlmittel aus dem Kühlmittelzufluß 14 in die Kühlmittelzuführung 16 und daraus in den Kühlmittelkanal 18 eintreten kann.

An seiner dem Bohrerschaft 22 zugewandten Ende weist der Kühlmittelzufluß 16 eine axiale Öffnung auf, die vollständig mit der axialen Öffnung des Kühlmittelkanals 18 des Bohrerschafts überlappt. Der Durchmesser der axialen Öffnung ist dabei geringfügig größer als die axiale Öffnung der Kühlmittelzuführung 16.

Der Bohrerschaft 22 und/oder die Kühlmittelzuführung 16 können auch an ihren Stoß- bzw. Stirnflächen so ausgebildet, daß kein zusätzliches Dichtungselement erforderlich ist, um eine ausreichende Abdichtung an der Grenzfläche zu erreichen. In diesem Fall sind die aufeinanderstoßenden Flächen des Bohrerschafts 22 und der Kühlmittelzuführung, genauer des Zwischenstücks 27 während des Betriebs selbstdichtend miteinander verbunden.

Insgesamt stellt sich ein im wesentlichen flüssigkeitsdichter Aufbau ein, in den beim Bohren erzeugte Knochenpartikel und Körperflüssigkeiten nicht eindringen können und außen auch nicht anhaften können, so daß eine einfache Reinigung ermöglicht ist.

Der Bohrerschaft 22 weist einen ersten rotationszylindrischen Abschnitt 23, welcher in das eigentliche Bohrwerkzeug übergeht, einen an diesen ersten rotationszylindrischen Abschnitt auf der von der Bohrerspitze abgewandten Seite anschließenden, im Querschnitt sechskantigen Abschnitt 28 und einen sich bis zu dem in die Aufnahme einzuführenden Ende des Bohrerschafts 22 erstreckenden zweiten rotationszylindrischen Abschnitt 26 auf.

Beim erfindungsgemäßen Bohrer ist die Aufnahme dabei mit einem sechskantigen Abschnitt zur Aufnahme des sechskantigen Bohrerschaftabschnitts 28 ausgebildet. Am Bohrerschaft 22 ist dann wenigstens über einen Teilumfang des Schaftes eine erste rechteckige Nut 24 am Übergang zwischen dem sechskantigen Abschnitt 28 und dem zylindrischem Abschnitt 26 vorgesehen, die mit einer mit der ersten Nut am Bohrerschaft 22 fluchtenden zweiten Nut 34 an der Aufnahme korrespondiert.

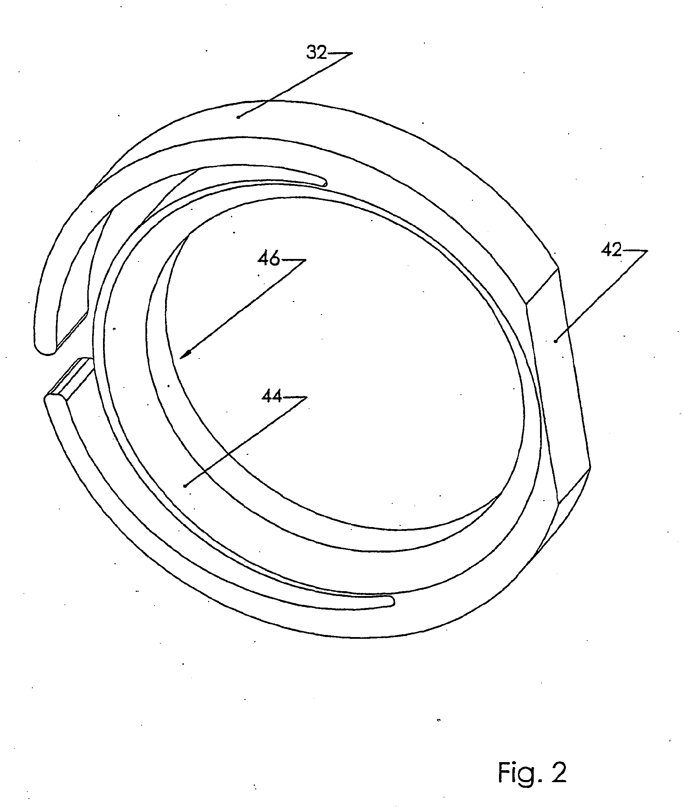

Die eigentliche Kupplungseinrichtung wird nun durch einen in die erste Nut-24 des Bohrerschafts 22 eingreifenden und in der zweiten Nut 34 des Anschlußstückes gehaltenen Federring 30 gebildet, der perspektivisch in der Fig. 2 dargestellt ist.

Grundsätzlich ist auch denkbar, den Bohrerschaft und die Aufnahme komplementär zur dargestellten Ausführungsform auszubilden, d.h. den Schaft mit einer Hülse zu versehen, die formschlüssig auf einen Dorn aufgesteckt wird. Diese Lösung ist allerdings aufwendig.

Der vorgeschlagene Federring 30 besteht aus einem geschlossenen Innenring 46 und wenigstens zwei freien Federarmen 32, die mit im wesentlichen gleichbleibender Stärke unter Belassung eines keilförmigen Freiraums zwischen den Federarmen 32 und dem Innenring 46, der sich zu dem Ansatzpunkt der Federarme 32 verjüngt, ausgebildet sind. Die Enden der Federarme 32 weisen aufeinander zu. Zwischen den Enden wird ein Abstand freigelassen, um den Federarmen ein federndes Aufeinanderzubewegen zu ermöglichen.

Die Arme des Federringes entspringen dabei jeweils der den Enden der Federarme gegenüberliegenden Hälfte des Innenrings 46, so daß mehr als der halbe Umfang durch die vorgelagerten Federarme eingenommen wird, während die Hälfte, an der die Arme angesetzt sind, in einer weiter bevorzugten Ausführung mit einem mit planer sekantenartiger Außenfläche 42 versehenen Segment ausgebildet ist, das an den Federarmen, genauer der Öffnung, die zwischen den Enden der Federarme verbleibt, gegenüber liegt.

Beim Einführen des Bohraufsatzes ist der Innenringinnenumfang des Federring 30 in seine konzentrische Stellung zum Hindurchlassen des Bohrerschafts 22 zu verschieben. Zum besseren Einführen des Schaftes ist der Federring 30 an der Ober- und/oder Unterseite mit einer abgeschrägten Kantenfläche 44 versehen.

Parallel zur Aufnahme 12 ist eine Außenhülse vorgesehen, die mit einem Druckelement entlang der Aufnahme verschieblich ist, um mit einer Betätigungszunge auf die in der Fig. 2 dargestellte Seitenfläche 42 des Federrings zu wirken, um diesen zum Lösen des Bohrerschafts 22 in eine konzentrische Stellung zu bringen, in der der Federing 30 nicht mehr in die erste Nut 24 eingreift und den Bohrerschaft 22 verriegelt. Hierfür weist das Druckelement eine abgeschrägte Anlagefläche auf.

Der neuartige Bohrerschaft ist in der Herstellung günstig, da sich die erste Nut 24 am Übergang zwischen dem mehrkantigen Abschnitt 28 und dem zylindrischen Abschnitt 26, die im radialen Querschnitt rechteckig, ansonsten aber radial umlaufend gebildet ist, in einem Arbeitsschritt mit dem Übergang zwischen kantigem und runden Abschnitt fertigen läßt.

Dabei kann die erste Nut 24 eine Tiefe aufweisen, die geringer als radiale Dicke des Innenrings 46 des Federrings 30 ist, so daß er in diese Nut - im Gegensatz zur Nut 34, die den Federring völlig aufnehmen kann - nie völlig einpaßt.

### Bezugszeichenliste

- 10: Anschlußstück
- 12: Aufnahme
- 14: Kühlmittelzufluß
- 16: Kühlmittelzuführung
- 18: Kühlmittelkanal
- 19: Ventil
- 20: Bohraufsatz
- 22: Bohrerschaft
- 23: erster zylindrischer Abschnitt
- 24: Nut
- 25: Rotationskupplung
- 26: zylindrischer Abschnitt
- 27: Zwischenstück
- 28: sechskantiger Abschnitt
- 30: Federring
- 32: Federarm
- 34: Nut

- 40: Dichtung
- 42: Außenfläche
- 44: Kantenfläche
- 46: Innenring

- 50: feststehender Teil der Rotationskupplung
- 52: rotierender Teil der Rotationskupplung
- 56: Feder
- 58: Ventilkopf
- 60: Ventilsitz
- 62: radiale Zuleitung

## Patentansprüche

1. Bohrer zum Erzeugen von Bohrungen in Knochengewebe mit einem Anschlussstück (10), in dem eine angetriebene Aufnahme (12) vorgesehen ist, und mit einem Bohraufsatz (20), der mit der Aufnahme (12) über eine Kupplungseinrichtung lösbar verbunden ist, wobei zumindest ein Abschnitt (28) eines Bohrerschafts (22) des Bohraufsatzes (20) in Mehrkantform ausgebildet ist und formschlüssig in einem komplementär ausgebildeten Bereich der Aufnahme (12) im Anschlussstück (10) aufgenommen ist, und ein distaler, im wesentlichen zylindrischer Abschnitt zum Bohren im Knochengewebe vorgesehen ist, wobei
der Bohrerschaft (22) mit einer ersten Nut (24) versehen ist,
die Aufnahme (12) mit einer mit der ersten Nut am Bohrerschaft (22) fluchtenden zweiten Nut (34) versehen ist,
und die Kupplungseinrichtung durch einen in die erste Nut (24) des Bohrerschafts (22) eingreifenden und in der zweiten Nut (34) des Anschlussstückes gehaltenen Federring (30) gebildet ist,
wobei der Federring (30) mit einem geschlossenen Innenring (46) und wenigstens zwei freien Federarmen (32) ausgebildet ist.

2. Bohrer nach Anspruch 1, **dadurch gekennzeichnet, dass** die Nut (24) am Übergang zwischen dem mehrkantigen Abschnitt (28) und einem zylindrischen Abschnitt (26) vorgesehen ist.

3. Bohrer nach Anspruch 1 oder 2, **dadurch gekennzeichnet, dass** die beiden Arme (32) des Federringes (30) im wesentlichen an einer Hälfte mit aufeinander zu weisenden Enden ausgebildet sind.

4. Bohrer nach einem der vorangehenden Ansprüche, **dadurch gekennzeichnet, dass** der Federring eine Flachseite aufweist, wobei der Innenumfang des Federrings mit einer zu dieser Flachseite abgeschrägten Kantenfläche (44) ausgebildet ist.

5. Bohrer nach einem der vorangehenden Ansprüche, **dadurch gekennzeichnet, dass** der Federring (30) mit einem mit gerader sekantenartiger Außenfläche (42) versehenen Segment ausgebildet ist, das den Federarmen gegenüber liegt.

6. Bohrer nach einem der Ansprüche 1 bis 6, **dadurch gekennzeichnet, dass** die erste Nut (24) als um den Schaft umlaufende radialsymmetrische Nut eine Tiefe aufweist, die geringer als die radiale Dicke des Innenrings (46) des Federrings (30) ist.

7. Verwendung eines Bohraufsatzes mit einem Bohrerschaft, der einen in Mehrkantformen ausgebildeten Abschnitt (28) aufweist, wobei unmittelbar angrenzend an diesen mehrkantigen Abschnitt eine Nut zur Aufnahme eines Federrings ausgebildet ist, zum Bilden eines Bohrers nach einem der Ansprüche 1 bis 7 durch formschlüssiges Aufnehmen des in Mehrkantform ausgebildeten Abschnitts des Bohraufsatzes in der komplementär ausgebildeten Aufnahme und lösbares Verbinden des Bohraufsatzes mit der Aufnahme über die Kupplungseinrichtung.

8. Verwendung eines Bohraufsatzes nach Anspruch 7, wobei bei dem Bohraufsatz die Nut am Übergang zwischen einem mehrkantigem Abschnitt (28) und einem rotationszylindrischen Abschnitt (26) des Bohraufsatzes vorgesehen ist.

9. Verwendung nach Anspruch 7 oder 8, wobei bei dem Bohraufsatz die Nut (24) über den radialen Querschnitt rechteckig ausgebildet ist.

## Claims

1. Drill for drilling holes in bone tissue, with an attachment piece (10), in which a driven holding fixture (12) is provided, and with a drill bit (20), which is detachably connected to the holding fixture (12) via a coupling device, at least one portion (28) of a drill shank (22) of the drill bit (20) being configured in a polygonal shape and being held with a form fit in the connecting piece (10) in an area of the holding fixture (12) that has a complementary shape, and a distal and substantially cylindrical portion being provided for drilling in the bone tissue, wherein the drill shank (22) is provided with a first groove (24), the holding fixture (12) is provided with a second groove (34) that is aligned with the first groove on the drill shank (22), the coupling device is formed by a spring ring (30), which engages in the first groove (24) of the drill shank (22) and which is held in the second groove (34) of the attachment piece, and the spring ring (30) is designed with a closed inner ring (46) and at least two free spring arms (32).

2. Drill according to Claim 1, **characterized in that** the groove (24) is provided at the transition between the polygonal portion (28) and a cylindrical portion (26).

3. Drill according to Claim 1 or 2, **characterized in that** the two arms (32) of the spring ring (30) are formed substantially on one half, with ends pointing towards each other.

4. Drill according to one of the preceding claims, **characterized in that** the spring ring has a flat side, the inner circumference of the spring ring being formed with an edge face (44) bevelled towards this flat side.

5. Drill according to one of the preceding claims, **characterized in that** the spring ring (30) is designed with a segment that is provided with a straight secant-like outer face (42) and that lies opposite the spring arms.

6. Drill according to one of Claims 1 to 5, **characterized in that** the first groove (24) extends radially symmetrically about the shank and has a depth that is less than the radial thickness of the inner ring (46) of the spring ring (30).

7. Use of a drill bit with a drill shank that has a polygonal portion (28), this polygonal portion being directly adjoined by a groove for receiving a spring ring, in order to form a drill according to one of Claims 1 to 6 by means of the polygonal portion of the drill bit being received with a form fit in the holding fixture of complementary shape and by means of the drill bit being connected detachably to the holding fixture via the coupling device.

8. Use of a drill bit according to Claim 7, the groove of the drill bit being provided at the transition between a polygonal portion (28) and a rotationally cylindrical portion (26) of the drill bit.

9. Use according to Claim 7 or 8, the groove (24) of the drill bit having a rectangular shape over the radial cross section.

## Revendications

1. Foret pour la réalisation de perçages dans un tissu osseux, comprenant un élément de raccordement (10) dans lequel est prévu un logement entraîné (12), et un embout de perçage (20), qui est relié de manière amovible au logement (12) par l'intermédiaire d'un dispositif d'accouplement, au moins une section (28) d'une tige de foret (22) de l'embout de perçage (20) ayant une forme polygonale et étant logée par correspondance de forme dans une zone du logement (12) réalisée de manière complémentaire, à l'intérieur de l'élément de raccordement (10), et une section distale essentiellement cylindrique étant prévue pour le perçage dans un tissu osseux,
la tige de foret (22) étant pourvue d'une première rainure (24),
le logement (12) étant pourvu d'une seconde rainure (34) en alignement avec la première rainure sur la tige de foret (22),
et le dispositif d'accouplement étant formé d'une rondelle élastique (30) s'engageant dans la première rainure (24) de la tige de foret (22) et maintenue dans la seconde rainure (34) de l'élément de raccordement,
la rondelle élastique (30) étant réalisée avec une bague intérieure fermée (46) et au moins deux bras élastiques libres (32).

2. Foret selon la revendication 1, **caractérisé en ce que** la rainure (24) est prévue au niveau de la transition entre la section polygonale (28) et une section cylindrique (26).

3. Foret selon la revendication 1 ou 2, **caractérisé en ce que** les deux bras (32) de la rondelle élastique (30) sont formés essentiellement sur une moitié avec deux extrémités orientées l'une vers l'autre.

4. Foret selon l'une quelconque des revendications précédentes, **caractérisé en ce que** la rondelle élastique présente un côté plat, le pourtour intérieur de la rondelle élastique étant réalisé avec une surface d'arête (44) chanfreinée vers ce côté plat.

5. Foret selon l'une quelconque des revendications précédentes, **caractérisé en ce que** la rondelle élastique (30) est réalisée avec un segment pourvu d'une surface extérieure (42) droite semblable à une sécante, ledit segment étant situé en face des bras élastiques.

6. Foret selon l'une quelconque des revendications 1 à 6, **caractérisé en ce que** la première rainure (24), en tant que rainure à symétrie radiale entourant la tige, présente une profondeur, qui est inférieure à l'épaisseur radiale de la bague intérieure (46) de la rondelle élastique (30).

7. Utilisation d'un embout de perçage comprenant une tige de foret, qui présente une section (28) polygonale, une rainure destinée à recevoir une rondelle élastique étant réalisée de manière directement contiguë à cette section polygonale afin de former un foret selon l'une quelconque des revendications 1 à 7 par réception, par correspondance de forme, de la section polygonale de l'embout de perçage dans le logement réalisé de manière complémentaire, et par liaison amovible de l'embout de perçage avec le logement par l'intermédiaire du dispositif d'accouplement.

8. Utilisation d'un embout de perçage selon la revendication 7, sachant que dans le cas de l'embout de perçage, la rainure est prévue au niveau de la transition entre une section polygonale (28) et une section cylindrique de révolution (26) de l'embout de perçage.

9. Utilisation selon la revendication 7 ou 8, sachant que dans le cas de l'embout de perçage, la rainure (24) est réalisée de manière rectangulaire sur la section radiale.
